# EUROPÄISCHE PATENTSCHRIFT

(11) **EP 2 789 519 B1**
(45) Veröffentlichungstag und Bekanntmachung des Hinweises auf die Patenterteilung: **28.12.2016**
(21) Anmeldenummer: 13163512.0
(22) Anmeldetag: 12.04.2013
(51) Int. Cl.: B61D 15/00, E01B 27/02

(54) **Vorrichtung zur Materialförderung im Gleisbau**
Device for transporting material in rail construction
Dispositif destiné au transport de matériaux dans la pose de voie

(43) Veröffentlichungstag der Anmeldung: 15.10.2014
(73) Patentinhaber: System7-Railsupport GmbH, 1010 Wien (AT)
(72) Erfinder: Lichtberger, Bernhard, 4230 Pregarten (AT)
(74) Vertreter: Patentanwaltskanzlei Hübscher

(56) Entgegenhaltungen:
- EP-A1- 0 303 037
- EP-A1- 1 300 313
- EP-A1- 1 384 815
- EP-A1- 2 360 316
- EP-A2- 2 194 189
- EP-A2- 2 500 470
- DE-B3-102009 037 568
- FR-A1- 2 930 502
- GB-A- 2 265 129

## Beschreibung

Die Erfindung bezieht sich auf eine Vorrichtung zur Materialförderung im Gleisbau, mit auf dem Gleis verfahrbaren Materialfördersiloeinheiten, die Material zwischen den Siloraum aufspannenden Seitenwänden auf einem Bodenförderband zur Förderung des Materials längs des Bodens aufnehmen, wobei ein Materialfördersilo mehrere aneinandergekuppelte Materialsiloeinheiten umfasst, die im Bereich ihrer einander zugewandten Stirnflächen mit Einrichtungen zur Übergabe des Materials von einer Siloeinheit zur nächsten Siloeinheit aufweisen, wobei der Materialfördersilo in Gelenkbauweise ausgeführt ist und wobei sich die Rahmen zweier aneinander anschließender Materialfördersiloeinheiten gelenkig auf einem gemeinsamen Laufwerk abstützen.

Derartige, aus der FR 2 930 502 A1, der GB 2 265 129 A und der EP 1 300 313 A1 bekannte Material-Förder-Siloeinheiten zum Laden, Liefern und Durchfördern von Gleisbaumaterial wie Schotter, Abraum oder Planumschutzschichtmaterial etc., finden im Gleisbau bzw. bei der Gleiswartung Verwendung. Die Materialfördersiloeinheiten werden bei Bau- und Umbaumaßnahmen für die Gleise der Eisenbahn eingesetzt. Sie befördern und speichern entweder Neumaterialien wie Schotter oder Planums-Schutzschichtmaterial oder Gebrauchsmaterialien wie Abraum der bei Schotterreinigungsarbeiten anfällt oder aber gereinigten Schotter. Sie werden auch als Zwischenspeicher bei Schotterverteilmaschinen o.ä. eingesetzt.

Bekannte Materialfördersiloeinheiten verfügen über Silowaggons, die Gleisbaumaterial zwischen den Siloraum aufspannenden Seitenwänden auf einem Bodenförderband zur Förderung des Materials längs des Bodens aufnehmen. Die Materialsiloeinheit umfasst mehrere Waggons, die im Bereich ihrer einander zugewandten Stirnwände mit einer Einrichtung zur Übergabe des Materials von einem Wagen zu dem nächsten Wagen ausgestattet sind (DE 10 2009 037 568 B3). Dazu sind beide Stirnwände jedes Waggons nach unten verschwenkbar und ist auf oder in den beiden Stirnwänden jeweils ein Förderband zur Übergabe des Materials von einem Wagen zum benachbarten Wagen vorgesehen. Nach einer anderen Ausgestaltungsvariante kann der Wagen an seinen beiden Kopfenden jeweils ein unteres Übergabeförderband im Bereich des Bodenförderbandes aufweisen, welches untere Übergabeförderband derart ansteuerbar und betätigbar ist, dass es ausgehend von einer horizontalen Ausgangsstellung nach oben in eine Abwurfstellung zur Übergabe des Materials an den benachbarten Wagen verschwankbar oder abknickbar ist.

Die Materialfördersiloeinheiten werden über bei der Eisenbahn bekannte Zugs-Stoßvorrichtungen gekuppelt und sind in verschiedenen Ausführungen bekannt. Es gibt kleinere Materialfördersiloeinheiten mit zwei zweiachsigen Drehgestellen, etwas größere mit zwei Doppeldrehgestellen (mit jeweils vier Achsen). Die Fahrzeuge sind als Selbstfahrer erhältlich oder in herkömmlicher Weise an einen Triebwagen kuppelbar. Zudem sind Ausführungen bekannt die zusätzlich auf Raupen, also gleislos, fahren können.

Da die Bodenförderbänder beim Stand der Technik jeweils auf das Übergabeförderband der nächsten Materialfördersiloeinheit fördern müssen, sind die Silos in Gleislängsrichtung leicht längsgeneigt bzw. erhöht angebracht um eine saubere Materialübergabe von einem Förderband auf das Nächste zu gewährleisten. Durch diese Längsneigung passt das Übergabeförderband unter das Bodenförderband. Nachteile dieser Ausführung sind der ungenutzte Raum unter der Schrägneigung bzw. Erhöhung und die erforderliche erhöhte Förderbandantriebskraft, die notwendig ist um das Fördermaterial die Neigung hinaufzubefördern. Nachteilig bei den vorhandenen Ausführungen ist auch, dass zwischen den einzelnen gekuppelten Materialfördersiloeinheiten ein relativ großer Leerraum im Bereich des Übergabeförderbandes entsteht. Dadurch ergeben sich relativ große Eigengewichte und verringerte nutzbare Ladevolumina. Dies bedeutet gleichzeitig, dass relativ viele Achsen pro nutzbarem Verladegewicht benötigt werden. In Europa ist die maximale Achslast derzeit mit 22,5 Tonnen begrenzt. Auch das Metergewicht mit welchem das Gleis belastet werden darf ist durch entsprechende Vorschriften begrenzt. Sollen die aus Materialfördersiloeinheiten zusammengestellten Züge auf Altstrecken verwendet werden, die wegen der Ausführung des Oberbaues und der Brücken nur geringere Achslasten und bestimmte Achsfolgen erlauben, ergeben sich erhebliche Einschränkungen zur Benutzung derselben. Die bei den am häufigsten eingesetzten Materialfördersiloeinheiten üblichen Doppeldrehgestelle sind überdies in der Ausführung relativ kompliziert und teuer. Wegen der Begrenztheit durch den länderabhängigen Lichtraum nach oben und seitlich ist der Nutzungsbereich der Silos räumlich stark eingeschränkt. Kurze Waggonlängen verringern das nutzbare Verladegewicht je Materialfördersiloeinheit zusätzlich.

Der Erfindung liegt somit die Aufgabe zugrunde, eine Vorrichtung der eingangs geschilderten Art zu schaffen, welche die vorgenannten Nachteile vermeidet, also insbesondere ein erhöhtes Nutzvolumen je Materialfördersiloeinheit aufweist, möglichst einfach aufgebaut ist und auch bei kleinen Längen je Materialfördersiloeinheit ein hohes nutzbares Verladegewicht je Materialfördersiloeinheit bereitstellt.

Die Erfindung löst die gestellte Aufgabe dadurch, dass der Siloraum durchgehend mit über die Materialfördersilolänge zumindest annähernd gleichem Siloraumquerschnitt, ausgeführt ist.

Erfindungswesentlich ist die Zusammenstellung des Materialfördersilos aus in Gelenkbauweise gekuppelten Materialfördersiloeinheiten. Dabei können im Prinzip beliebig viele einzelne Materialfördersiloeinheiten miteinander verbunden werden. Die einzelnen Materialfördersiloeinheiten werden gelenkig miteinander verbunden, wodurch ein zumindest annähernd gleicher Siloraumquerschnitt über die Materialfördersilolänge gegeben ist und ein maximales Nutzvolumen je Materialfördersiloeinheit zur Verfügung steht. Damit und da sich die Rahmen zweier aneinander anschließender Materialfördersiloeinheiten gelenkig auf einem gemeinsamen Laufwerk abstützen steht auch bei kleinen Längen je Materialfördersiloeinheit ein hohes nutzbares Verladegewicht (Ladevolumen) je Materialfördersiloeinheit zur Verfügung. Die Länge der Materialfördersiloeinheiten ergibt sich aus den Randbedingungen, wie maximale Radlasten, Radlastfolgen, Lichtraumprofil und Kurvenradien.

Das Drehgelenk sitzt direkt über dem Laufwerk, beispielsweise einem Drehgestell. Über eine Drehscheibe oder über Übergangsplatte wird der offene Bereich zwischen den Förderbändern die auf gleicher Höhe (ausgenommen der Übergabeteil) überbrückt. Insbesondere sind im Bodenbereich über dem Laufwerk und zwischen den Bodenförderbändern zweier aneinander anschließender Materialfördersiloeinheiten Längsneigungs- und Drehausgleichsborde vorgesehen. Durch die von den Förderbändern auf das aufgeschüttete Material ausgeübte Kraft gleitet das Material über die Drehscheibe bzw. Längsneigungs- und Drehausgleichsborde, die auch mit einem Vibrationsantrieb ausgestattet sein können, damit der Transport über sie leichter erfolgen kann. Es empfiehlt sich die Längsneigungs- und Drehausgleichsborde aus abscherfestem sehr gleitfähigem Material, wie beispielsweise kohlenstofffaserverstärkter Kunststoff, zu fertigen.

Dazu empfiehlt es sich, wenn den Längsneigungs- und Drehausgleichsborden ein Stelltrieb zugeordnet ist, mit dem diese beim Entladen aus einer liegenden Arbeitsstellung in eine aufgestellte Abladestellung anhebbar sind, um dem abtransportierenden Bodenförderband das nach dem Entladen noch auf den Längsneigungs- und Drehausgleichsborden aufliegende Restmaterial zuzuführen. Die Längsneigungs- und Drehausgleichsborde werden also so ausgeführt, dass diese beim Entladen angehoben werden können, um Restmaterial, das nach dem Entladen noch immer darauf aufliegt ebenfalls auf das abtransportierende Förderband aufladen zu können. Zudem bzw. alternativ können die Längsneigungs- und Drehausgleichsborde als Schwingförderer ausgebildet sein, mit welchen Schwingförderern auf den Längsneigungs- und Drehausgleichsborden aufliegendes Material einem abtransportierenden Bodenförderband zuführbar ist. Die Längsneigungs- und Drehausgleichsborde bewegen sich schräg nach oben in Transportrichtung und zurück, wobei das Fördergut abhängig von der Wurfkennziffer zur Förderung angehoben und in die gewollte Richtung geworfen wird. Beim Rückhub bleibt das Fördergut massenträgheitsbedingt stehen. Dieser Schwingfördererantrieb dient primär dazu auf den Längsneigungs- und Drehausgleichsborden aufliegendes Restmaterial anzutransportieren.

Damit sich die Materialfördersiloeinheiten um die Silolängsachse nicht gegeneinander verwinden können, sind die Rahmen zweier aneinander anschließender, auf einem gemeinsamen Laufwerk abgestützter Materialfördersiloeinheiten mit einer eine freie gegenseitige Torsionsbewegung der beiden Rahmenteile um die Vorrichtungslängsachse unterbindenden Verwindungssperre ausgestattet. Besonders einfach und vorteilhaft kann eine derartige Verwindungssperre realisiert werden, wenn die Verwindungssperre wenigstens einen von einer Rahmenflanke in Vorrichtungslängsrichtung gegen den Rahmen der anschließenden Materialfördersiloeinheit vorragenden Rahmenfortsatz umfasst, der in eine entsprechende gegengleiche Führungsaufnahme eingreift.

Beispielsweise ragen vom Rahmen aus links und rechts des Laufwerkes Rahmenfortsätze vor, die in entsprechenden Gabelstückes am benachbarten Rahmen geführt sind. Zur Führung der Rahmenfortsätze sind beispielsweise seitlich, oberhalb und unterhalb der Rahmenfortsätze bombierte Rollen in den Gabelstücken gelagert. Die Toleranzen sind derart zu bemessen, dass die notwendigen horizontalen und vertikalen Drehungen der gekuppelten Materialfördersiloeinheiten ohne Verspannungen möglich sind, eine freie Bewegung um die Materialfördersilolängsächse aber unterbunden wird. Die Entgleisungssicherheit wird von den Federungen der Laufwerke, und den Spielen in der Auflage der Wagenteile am Laufwerk sichergestellt.

Um den Silo im Bereich der Drehgelenke seitenwandig zwischen benachbarten Materialfördersiloeinheiten vorteilhaft zu schließen wird vorgeschlagen, dass den Seitenwänden stirnseitig gegen die anschließende Materialfördersiloeinheit vorragende, beweglich an den Seitenwänden angeordnete Längsausgleichsplatten zugeordnet sind, wobei sich die einander zugeordneten Längsausgleichsplatten und gegebenenfalls Seitenwände aneinander anschließender Materialfördersiloeinheiten in Vorrichtungslängsrichtung überlappen. Beispielsweise können einander zugeordneten Längsausgleichsplatten in Vorrichtungslängsrichtung einerends an einer Seitenwand gelenkig gelagert und anderends entlang der Seitenwand der anschließenden Materialfördersiloeinheit verschiebbar geführt sein. Die Längsneigungs- und Drehausgleichsborde und/oder die Längsausgleichsplatten bestehen vorzugsweise aus besonders verschleißfestem Material mit geringem Reibwert, nämlich aus Kohlefaserverbundwerkstoffen, aus hartbeschichteten Stahlblechen oder aus Hardox-Blechen.

Das Laufwerk kann Einzelachsen, zweiachsige oder dreiaclisige Drehgestelle oder Doppeldrehgestelle umfassen. Zudem weist das Laufwerk oberseitig einen Anschlag für von den Rahmen zweier aneinander anschließender Materialfördersiloeinheiten gegeneinander vorragender Kupplungsansätze auf, wobei insbesondere einer der beiden Kupplungsansätze den anderen gabelförmig einfasst und beide Kupplungsansätze gemeinsam am Anschlag angreifen. Eine derartige Ausführung der Verbindung erlaubt freie horizontale und vertikale Verdrehungen der beiden Rahmenteile zueinander, womit sich der Verbund aus den Materialfördersiloeinheiten Bögen und Neigungswechseln anpassen kann.

Im Gegensatz zum Stand der Technik, der für jeden einzelnen Waggon ein Dieselaggregat, einen Generator und ein Hydrauliksystem vorsieht, ist bei der erfindungsgemäßen Vorrichtung vorzugsweise nur ein Dieselmotor, ein Stromgenerator und eine Steuerung zur Versorgung und Ansteuerung der Hydraulikanlagen aller Materialfördersiloeinheiten vorgesehen.

Der Materialfördersilo kann sowohl mit Laufachsen als auch als Selbstfahreinheit ausgeführt werden. Ebenso besteht die Möglichkeit den Materialfördersilo zusätzlich als gleislos fahrbares Fahrzeug auszuführen, wozu unterhalb am Rahmen zusätzlich angebrachte Raupen vorgesehen werden können.

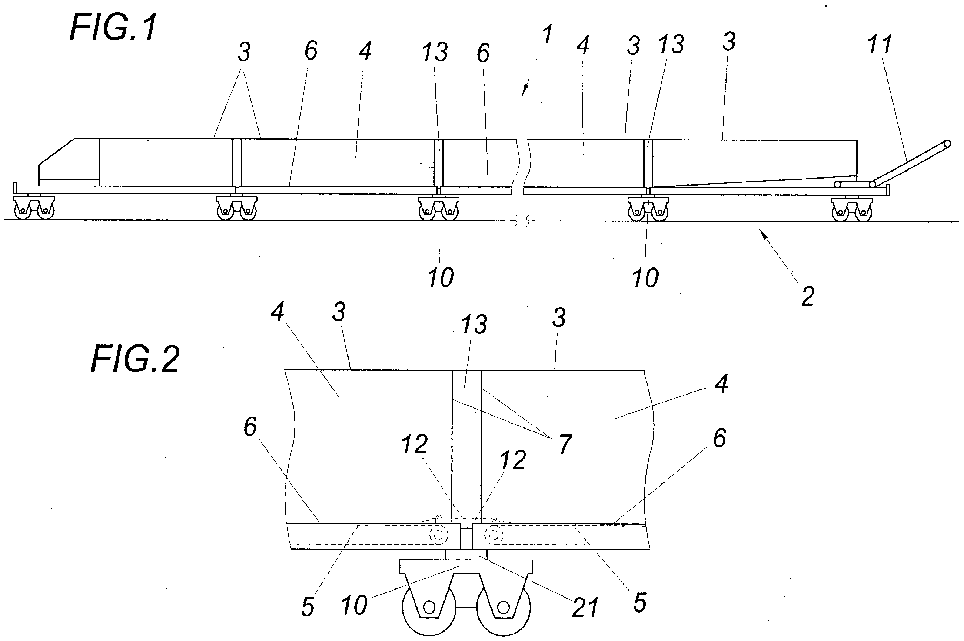

In der Zeichnung ist der Erfindungsgegenstand beispielsweise dargestellt. Es zeigen
- Fig. 1: einen erfindungsgemäßen Materialfördersilo in teilgeschnittener Seitenansicht,
- Fig. 2: einen vergrößerten Ausschnitt aus Fig. 1 im Bereich einer Laufachse,

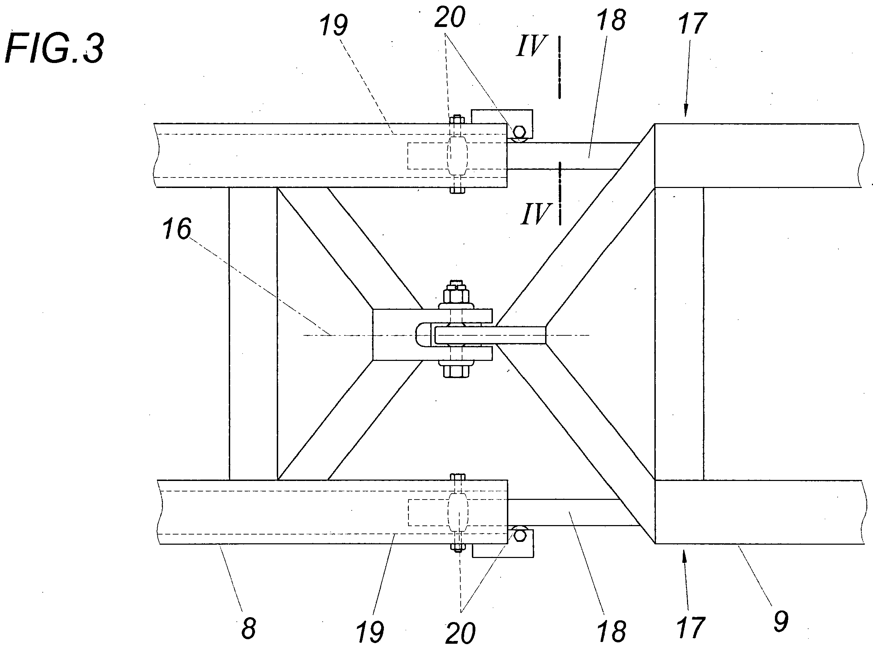

- Fig. 3: eine Draufsicht auf zwei aneinander anschließende Materialfördersiloeinheitsrahmen im Kupplungsbereich,

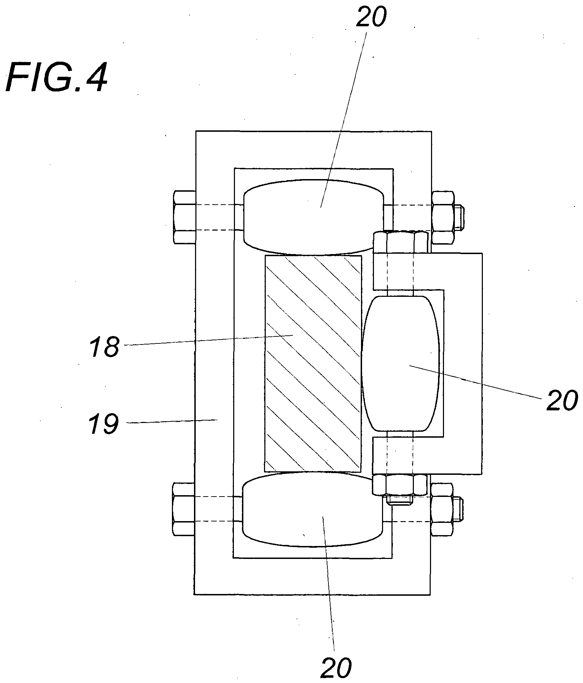

- Fig. 4: die Führung aus Fig.3 im Schnitt nach der Linie IV-IV und in vergrößerter Darstellung,

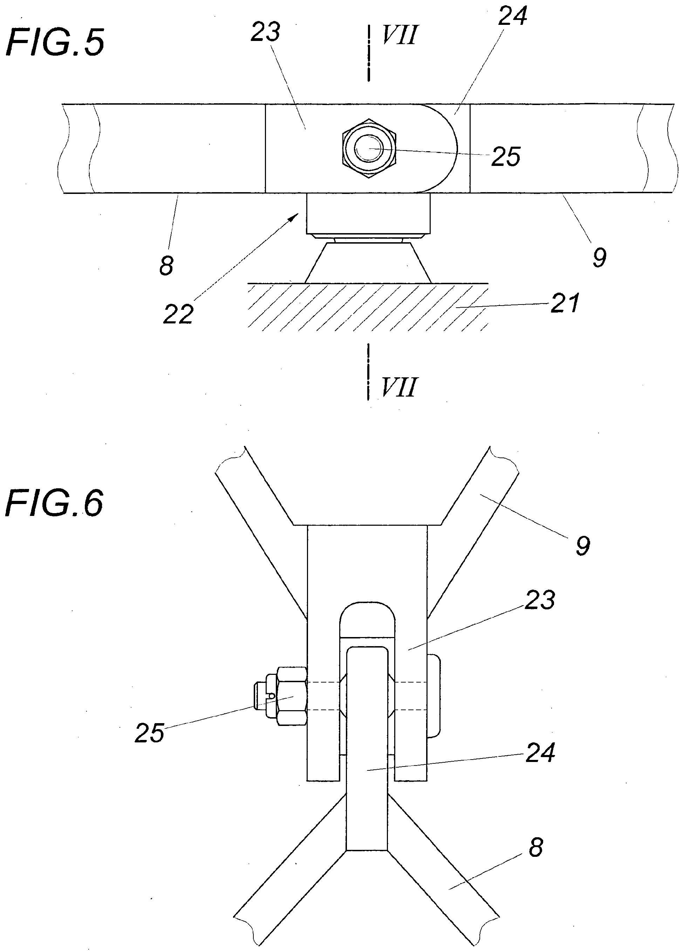

- Fig. 5: die vergrößerte Kupplung aus Fig. 3 in Seitenansicht,
- Fig. 6: die vergrößerte Kupplung aus Fig. 3 in Draufsicht,

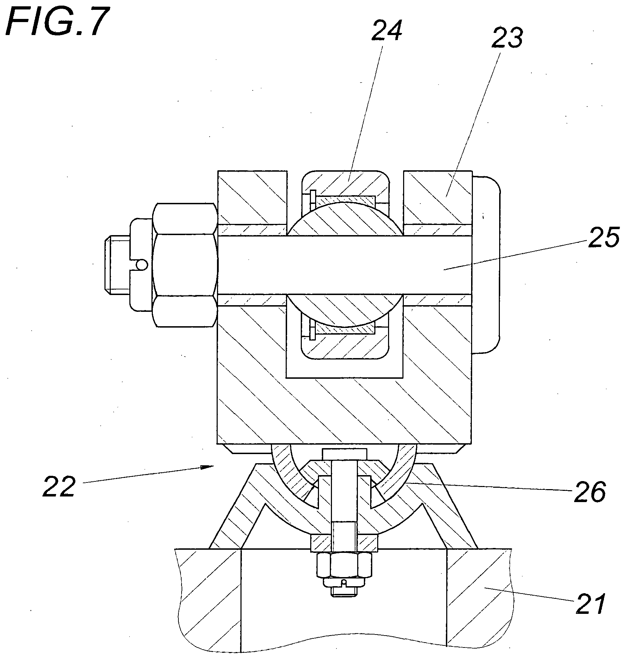

- Fig. 7: die Kupplung aus Fig. 5 in Schnitt nach der Linie VII-VII und

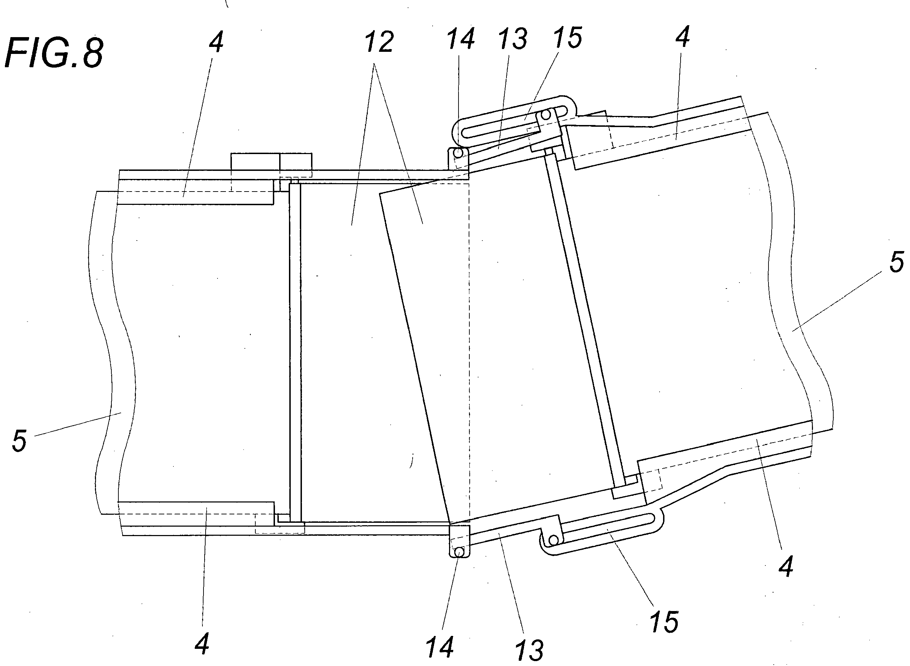

- Fig. 8: eine vereinfachte Draufsicht auf die Vorrichtung aus Fig. 1.

Die dargestellte Vorrichtung zur Materialförderung im Gleisbau, ein Materialfördersilo 1, umfasst auf einem Gleis 2 verfahrbare Materialfördersiloeinheiten 3, die Material zwischen den Siloraum aufspannenden Seitenwänden 4 auf einem Bodenförderband 5 zur Förderung des Materials längs des Bodens 6 aufnehmen, wobei ein Materialfördersilo 1 mehrere aneinandergekuppelte Materialsiloeinheiten 3 umfasst, die im Bereich ihrer einander zugewandten Stirnflächen 7 mit Einrichtungen zur Übergabe des Materials von einer Materialfördersiloeinheit 3 zur nächsten Materialfördersiloeinheit 3 aufweisen.

Zur bestmöglichen Ausnutzung des theoretisch zur Verfügung stehenden Silogesamtvolumens ist der Materialfördersilo 1 in Gelenkbauweise ausgeführt, wobei sich die Rahmen 8, 9 zweier aneinander anschließender Materialfördersiloeinheiten 3 gelenkig auf einem gemeinsamen Laufwerk 10 abstützen und der Siloraum durchgehend, insbesondere mit über die Materialfördersiloeinheitslänge zumindest annähernd gleichem Siloraumquerschnitt, ausgeführt ist. Lediglich an der Zugspitze bzw. am Zugende sind herkömmliche Übergabevorrichtungen 11 vorgesehen, um den Zug mit bestehenden Systemen verwenden zu können.

Im Bodenbereich über dem Laufwerk 10 und zwischen den Bodenförderbändern 5 zweier aneinander anschließender Materialfördersiloeinheiten 3 sind Längsneigungs- und Drehausgleichsborde 12 vorgesehen, über welche das Material bei einem Betrieb der Bodenförderbänder 5 geschoben wird. Den Längsneigungs- und Drehausgleichsborden 12 ist ein Stelltrieb zugeordnet, mit dem diese beim Entladen aus einer liegenden Arbeitsstellung in eine aufgestellte Abladestellung anhebbar sind, um dem abtransportierenden Bodenförderband 5 das nach dem Entladen noch auf den Längsneigungs- und Drehausgleichsborden 12 aufliegende Restmaterial zuzuführen.

Zum Abdichten der Seitenwände 4 im Bereich zwischen den Materialfördersiloeinheiten 3 über den Drehgelenken sind den Seitenwänden 4 stirnseitig gegen die anschließende Materialfördersiloeinheit 3 vorragende, beweglich an den Seitenwänden 4 angeordnete Längsausgleichsplatten 13 zugeordnet, wobei sich die einander zugeordneten Längsausgleichsplatten 13 und gegebenenfalls Seitenwände 4 aneinander anschließender Materialfördersiloeinheiten 3 in Vorrichtungslängsrichtung überlappen. Die einander zugeordneten Längsausgleichsplatten 13 sind in Vorrichtungslängsrichtung einerends an einer Seitenwand 4 in Lagern 14 gelenkig gelagert und anderends in einer Führung 15 entlang der Seitenwand 4 der anschließenden Materialfördersiloeinheit 3 verschiebbar geführt.

Zudem sind die Rahmen 8, 9 zweier aneinander anschließender auf einem gemeinsamen Laufwerk 10 aufruhender Materialfördersiloeinheiten 3 mit einer eine freie gegenseitige Torsionsbewegung der beiden Rahmenteile 8, 9 um die Vorrichtungslängsachse 16 unterbindenden Verwindungssperre ausgestattet. Die Verwindungssperre umfasst zwei von je einer Rahmenflanke 17 in Vorrichtungslängsrichtung 16 gegen den Rahmen 8 der anschließenden Materialfördersiloeinheit 3 vorragende Rahmenfortsätze 18, die in entsprechende gegengleiche Führungsaufnahmen 19 eingreifen. Zur Führung der Rahmenfortsätze 18 in den Führungsaufnahmen 19 sind seitlich, oberhalb und unterhalb der Rahmenfortsätze 18 bombierte Rollen 20 in den Führungsaufnahmen 19 gelagert.

Das Laufwerk 10 ist im Ausführungsbeispiel zweiachsig ausgebildet und umfasst oberseitig an einem Fahrschemel 21 einen Anschlag 22 für von den Rahmen 8, 9 zweier aneinander anschließender Materialfördersiloeinheiten 3 gegeneinander vorragender Kupplungsansätze 23, 24 umfasst. Dabei fasst der Kupplungsansatz 23 den anderen Kupplungsansatz 24 gabelförmig ein und greifen beide Kupplungsansätze 23, 24 gemeinsam am Anschlag 22 an. Die beiden Kupplungsansätze 23 und 24 sind mit einem Kuppelbolzen 25 am Anschlag 22 befestigt, der wegen einer Kugelkupplung zwei Freiheitsgrade der Rotation ermöglicht. Die Gabel des Kupplungsansatzes 23 ist unterseitig mit einer Drehpfanne 26 verbunden die mit dem Fahrschemel 21 (dem Obergurt) des Laufwerkes 10 verbunden ist.

Diese Ausführung der Kupplung erlaubt eine horizontale und eine vertikale Verdrehung der beiden Rahmenteile 8, 9 zueinander, womit die sich die Materialfördersiloeinheiten den Bögen und den Neigungswechseln des Gleises frei anpassen können.

## Patentansprüche

1. Vorrichtung zur Materialförderung im Gleisbau, mit auf einem Gleis (2) verfahrbaren Materialfördersiloeinheiten (3), die Material zwischen den Siloraum aufspannenden Seitenwänden (4) auf einem Bodenförderband (5) zur Förderung des Materials längs des Bodens (6) aufnehmen, wobei ein Materialfördersilo (1) mehrere aneinandergekuppelte Materialsiloeinheiten (3) umfasst, die im Bereich ihrer einander zugewandten Stirnflächen (7) mit Einrichtungen zur Übergabe des Materials von einer Materialfördersiloeinheit (3) zur nächsten Materialfördersiloeinheit (3) aufweisen, wobei der Materialfördersilo (1) in Gelenkbauweise ausgeführt ist und wobei sich die Rahmen (8, 9) zweier aneinander anschließender Materialfördersiloeinheiten (3) gelenkig auf einem gemeinsamen Laufwerk (10) abstützen, **dadurch gekennzeichnet, dass** der Siloraum durchgehend, mit über die Materialfördersilolänge zumindest annähernd gleichem Siloraumquerschnitt, ausgeführt ist.

2. Vorrichtung nach Anspruch 1, **dadurch gekennzeichnet, dass** im Bodenbereich über dem Laufwerk (10) und zwischen den Bodenförderbändern (5) zweier aneinander anschließender Materialfördersiloeinheiten (3) Längsneigungs- und Drehausgleichsborde (12) vorgesehen sind, die den offenen Bereich zwischen den Förderbändern auf gleicher Höhe überbrücken.

3. Vorrichtung nach Anspruch 2, **dadurch gekennzeichnet, dass** den Längsneigungs- und Drehausgleichsborden (12) ein Stelltrieb zugeordnet ist, mit dem diese beim Entladen aus einer liegenden Arbeitsstellung in eine aufgestellte Abladestellung anhebbar sind, um dem abtransportierenden Bodenförderband (5) das nach dem Entladen noch auf den Längsneigungs- und Drehausgleichsborden (12) aufliegende Restmaterial zuzuführen.

4. Vorrichtung nach Anspruch 2 oder 3, **dadurch gekennzeichnet, dass** die Längsneigungs- und Drehausgleichsborde (12) als Schwingförderer ausgebildet sind, mit dem auf den Längsneigungs- und Drehausgleichsborden (12) aufliegendes Material einem abtransportierenden Bodenförderband (5) zuführbar ist.

5. Vorrichtung nach einem der Ansprüche 1 bis 4, **dadurch gekennzeichnet, dass** die Rahmen (8, 9) zweier aneinander anschließender auf einem gemeinsamen Laufwerk (10) aufruhender Materialfördersiloeinheiten (3) mit einer eine freie gegenseitige Torsionsbewegung der beiden Rahmenteile (8, 9) um die Vorrichtungslängsachse (16) unterbindenen Vcrwindungssperre ausgestattet sind.

6. Vorrichtung nach Anspruch 5, **dadurch gekennzeichnet, dass** die Verwindungssperre wenigstens einen von einer Rahmenflanke (17) in Vorrichtungslängsrichtung (16) gegen den Rahmen (8) der anschließenden Materialfördersiloeinheit (3) vorragenden Rahmenfortsatz (18) umfasst, der in eine entsprechende gegengleiche Führungsaufnahme (19) eingreift.

7. Vorrichtung nach einem der Ansprüche 1 bis 6, **dadurch gekennzeichnet, dass** den Seitenwänden (4) stirnseitig gegen die anschließende Materialfördersiloeinheit (3) vorragende, beweglich an den Seitenwänden (4) angeordnete Längsausgleichsplatten (13) zugeordnet sind, wobei sich die einander zugeordneten Längsausgleichsplatfen (13) und gegebenenfalls Seitenwände (4) aneinander anschließender Materialfördersiloeinheiten (3) in Vorrichtungslängsrichtung überlappen.

8. Vorrichtung nach Anspruch 7, **dadurch gekennzeichnet, dass** die einander zugeordneten Längsausgleichsplatten (13) in Vorrichtungslängsrichtung einerends an einer Seitenwand (4) gelenkig gelagert und anderends entlang der Seitenwand (4) der anschließenden Materialfördersiloeinheit (3) verschiebbar geführt sind.

9. Vorrichtung nach einem der Ansprüche 2 bis 8, **dadurch gekennzeichnet, dass** die Längsneigungs- und Drehausgleichsborde (12) und/oder die Längsausgleichsplatten (13) aus besonders verschleißfestem Material mit geringem Reibwert, nämlich aus Kohlefaserverbundwerkstoffen, aus hartbeschichteten Stahlblechen oder aus Hardox-Blechen bestehen.

10. Vorrichtung nach einem der Ansprüche 1 bis 9, **dadurch gekennzeichnet, dass** das Laufwerk (10) Einzelachsen, zweiachsige oder dreiachsige Drehgestelle oder Doppeldrehgestelle umfasst und oberseitig einen Anschlag für von den Rahmen zweier aneinander anschließender Materialfördersiloeinheiten gegeneinander vorragender Kupplungsansätze umfasst, wobei insbesondere einer der beiden Kupplungsansätze den anderen gabelförmig einfasst und beide Kupplungsansätze gemeinsam am Anschlag angreifen.

11. Vorrichtung nach einem der Ansprüche 1 bis 10, **dadurch gekennzeichnet, dass** ein Dieselmotor, ein Stromgenerator und eine Steuerung zur Versorgung und Ansteuerung der Hydraulikanlagen aller Materialfördersiloeinheiten (3) vorgesehen ist.

## Claims

1. Apparatus for conveying material during track construction, comprising material conveyance silo units (3) which can be moved on a track (2) and which receive material between sidewalls (4), which span the silo chamber, on a bottom conveyor belt (5) for conveying the material along the bottom (6), wherein a material conveyance silo (1) comprises a plurality of material silo units (3) which are coupled together and which in the region of the mutually facing end surfaces (7) thereof comprise devices for transferring the material from one material conveyance silo unit (3) to the next material conveyance silo unit (3), wherein the material conveyance silo (1) is of articulated design and wherein the frames (8, 9) of two mutually adjoining material conveyance silo units (3) are supported in an articulated manner on a common running gear (10), **characterised in that** the silo chamber is of continuous design with an at least approximately identical silo chamber cross-section over the material conveyance silo length.

2. Apparatus as claimed in claim 1, **characterised in that** longitudinal inclination and rotational equalisation boards (12) are provided in the bottom region above the running gear (10) and between the bottom conveyor belts (5) of two mutually adjoining material conveyance silo units (3), which boards bridge the open region between the conveyor belts at the same height.

3. Apparatus as claimed in claim 2, **characterised in that** the longitudinal inclination and rotational equalisation boards (12) are allocated an actuator, by means of which said boards can be raised, during unloading, from a horizontal operating position to a deployed unloading position, in order to supply the remaining material, which is still lying on the longitudinal inclination and rotational equalisation boards (12) after unloading, to the bottom conveyor belt (5) used for removal purposes.

4. Apparatus as claimed in claim 2 or 3, **characterised in that** the longitudinal inclination and rotational equalisation boards (12) are designed as a vibration conveyor, by means of which material lying on the longitudinal inclination and rotational equalisation boards (12) can be supplied to a bottom conveyor belt (5) used for removal purposes.

5. Apparatus as claimed in any one of claims 1 to 4, **characterised in that** the frames (8, 9) of two mutually adjoining material conveyance silo units (3), which rest on a common running gear (10), are equipped with an anti-twist mechanism which prevents free mutual torsional movement of the two frame parts (8, 9) about the longitudinal axis (16) of the apparatus.

6. Apparatus as claimed in claim 5, **characterised in that** the anti-twist mechanism comprises at least one frame extension (18) which protrudes from a frame flank (17) in the longitudinal direction (16) of the apparatus against the frame (8) of the adjoining material conveyance silo unit (3) and engages into a corresponding mirror-inverted guide receiver (19).

7. Apparatus as claimed in any one of claims 1 to 6, **characterised in that** on the end side the sidewalls (4) are allocated longitudinal equalisation plates (13) which protrude against the adjoining material conveyance silo unit (3) and are arranged to be movable on the sidewalls (4), wherein the mutually allocated longitudinal equalisation plates (13) and optionally sidewalls (4) of mutually adjoining material conveyance silo units (3) overlap in the longitudinal direction of the apparatus.

8. Apparatus as claimed in claim 7, **characterised in that** at one end the mutually allocated longitudinal equalisation plates (13) are mounted in articulated manner on a sidewall (4) in the longitudinal direction of the apparatus and at the other end are guided so as to be displaceable along the sidewall (4) of the adjoining material conveyance silo unit (3).

9. Apparatus as claimed in any one of claims 2 to 8, **characterised in that** the longitudinal inclination and rotational equalisation boards (12) and/or the longitudinal equalisation plates (13) consist of particularly wear-resistant material having a low coefficient of friction, namely carbon fibre composite materials, hard-coated steel plates or Hardox plates.

10. Apparatus as claimed in any one of claims 1 to 9, **characterised in that** the running gear (10) comprises single axles, two-axle or three-axle bogies or twin-bogies and comprises on the top side a stop for coupling lugs which protrude against one another from the frames of two mutually adjoining material conveyance silo units, wherein in particular one of the two coupling lugs surrounds the other in a forked manner and both coupling lugs act together upon the stop.

11. Apparatus as claimed in any one of claims 1 to 10, **characterised in that** a diesel engine, a current generator and a controller are provided to power and activate the hydraulic systems of all of the material conveyance silo units (3).

## Revendications

1. Dispositif pour le transport de matériaux dans la pose de voies, avec des unités silo de transport de matériaux (3), déplaçables sur une voie (2), qui reçoivent du matériau entre les parois latérales (4) enserrant l'espace silo sur une bande transporteuse au fond (5) pour transporter le matériau le long du fond (6), un silo de transport de matériaux (1) comprenant plusieurs unités silo de transport de matériaux (3), accouplées les unes aux autres, qui présentent dans la zone de leurs faces frontales (7), tournées les unes vers les autres, des dispositifs pour transférer le matériau d'une unité silo de transport de matériaux (3) à la plus proche unité silo de transport de matériaux (3), le silo de transport de matériaux (1) étant réalisé en mode de structure articulée et les cadres (8, 9) de deux unités silo de transport de matériaux (3) adjacentes s'appuyant de manière articulée sur un organe de roulement (10) commun, **caractérisé en ce que** l'espace silo est réalisé de manière continue, avec une section transversale d'espace silo au moins approximativement identique sur la longueur du silo de transport de matériaux.

2. Dispositif selon la revendication 1, **caractérisé en ce que** des bords compensateurs de rotation et d'inclinaison longitudinale (12) sont prévus dans la zone de fond, au-dessus de l'organe de roulement (10) et entre les bandes transporteuses au fond (5) de deux unités silo de transport de matériaux (3) adjacentes qui couvrent la zone ouverte entre les bandes transporteuses sur la même hauteur.

3. Dispositif selon la revendication 2, **caractérisé en ce qu'**une commande de réglage est associée aux bords compensateurs de rotation et d'inclinaison longitudinale (12) avec laquelle ceux-ci peuvent être soulevés lors du déchargement depuis une position de travail horizontale à une position de déchargement donnée, afin d'alimenter la bande transporteuse au fond (5) transportant, en matériau reposant encore, après le déchargement, sur les bords compensateurs de rotation et d'inclinaison longitudinale (12).

4. Dispositif selon la revendication 2 ou 3, **caractérisé en ce que** les bords compensateurs de rotation et d'inclinaison longitudinale (12) sont conçus en tant que transporteur à vibrations, avec lequel le matériau reposant sur les bords compensateurs de rotation et d'inclinaison longitudinale (12) peut être amené à une bande transporteuse au fond (5) transportant.

5. Dispositif selon l'une des revendications 1 à 4, **caractérisé en ce que** les cadres (8, 9) de deux unités silo de transport de matériaux (3) adjacentes, reposant sur un organe de roulement commun (10), sont équipés d'un dispositif de blocage contre la distorsion empêchant tout mouvement de torsion réciproque libre des deux parties de cadre (8, 9) autour de l'axe longitudinal (16) du dispositif.

6. Dispositif selon la revendication 5, **caractérisé en ce que** le dispositif de blocage contre la distorsion comprend au moins un prolongement de cadre (18) en saillie d'un flanc de cadre (17) dans le sens longitudinal du dispositif (16) à l'opposé du cadre (8) de l'unité silo de transport de matériau (3) reliée, qui vient en prise dans un logement de guidage (19) correspondant et opposé.

7. Dispositif selon l'une des revendications 1 à 6, **caractérisé en ce que** des plaques de compensation longitudinale (13) disposées mobiles au niveau des parois latérales (4), sont associées en saillie des parois latérales (4), face avant contre l'unité silo de transport de matériaux (3) reliée, les plaques de compensation longitudinale (13) associées les unes aux autres, et, le cas échéant, les parois latérales (4) des unités silo de transport de matériaux (3) adjacentes, se chevauchant dans le sens longitudinal du dispositif.

8. Dispositif selon la revendication 7, **caractérisé en ce que** les plaques de compensation longitudinale (13) associées les unes aux autres dans le sens longitudinal du dispositif sont montées de manière articulée, à une extrémité, au niveau d'une paroi latérale (4) et, à l'autre extrémité, sont guidées, pouvant se déplacer, le long de la paroi latérale (4) de l'unité silo de transport de matériaux (3) reliée.

9. Dispositif selon l'une des revendications 2 à 8, **caractérisé en ce que** les bords compensateurs de rotation et d'inclinaison longitudinale (12) et/ou les plaques de compensation longitudinale (13) sont en matériau particulièrement résistant à l'usure avec un faible coefficient de frottement, à savoir en matériaux composites en fibre de carbone, en tôles d'acier à enduction dure ou en tôles Hardox.

10. Dispositif selon l'une des revendications 1 à 9, **caractérisé en ce que** l'organe de roulement (10) comprend des essieux uniques, des bâtis tournants à deux ou trois essieux ou des bâtis tournants doubles, et comprend en partie supérieure une butée pour des embouts d'accouplement dépassant réciproquement des cadres des deux unités silo de transport de matériaux adjacentes, notamment, un des deux embouts d'accouplement enserrant l'autre comme une fourche et les deux embouts d'accouplement étant en prise conjointement au niveau de la butée.

11. Dispositif selon l'une des revendications 1 à 10, **caractérisé en ce que** sont prévus un moteur diesel, un générateur de courant et une commande pour alimenter et piloter les installations hydrauliques de toutes les unités silo de transport de matériaux (3).
